# EUROPÄISCHE PATENTSCHRIFT

(11) **EP 3 000 063 B1**
(45) Veröffentlichungstag und Bekanntmachung des Hinweises auf die Patenterteilung: **18.12.2019**
(21) Anmeldenummer: 14724493.3
(22) Anmeldetag: 19.05.2014
(51) Int. Cl.: G16H 40/63, G16H 20/30

(54) **ABGABEVORRICHTUNG ZUR ABGABE EINES SPIELGEGENSTANDES**
LAUNCHING DEVICE FOR LAUNCHING A SPORTS OBJECT
DISPOSITIF LANCEUR DESTINÉ À ENVOYER UN OBJET DE JEU

(30) Priorität: 21.05.2013 DE 102013105164
(43) Veröffentlichungstag der Anmeldung: 30.03.2016
(73) Patentinhaber: Fraunhofer Gesellschaft zur Förderung der angewandten Forschung e.V., 80686 München (DE)
(72) Erfinder: GURZI, Domenico Francesco, 65185 Wiesbaden (DE)
(74) Vertreter: Patentship Patentanwaltsgesellschaft mbH
(86) Internationale Anmeldenummer: PCT/EP2014/060245
(87) Internationale Veröffentlichungsnummer: WO 2014/187776

(56) Entgegenhaltungen:
- JP-A- S5 734 877
- US-A1- 2013 018 493
- US-A1- 2013 104 869

## Beschreibung

Die vorliegende Erfindung betrifft das Gebiet des Sporttrainings mit Sportgegenständen.

Die Offenlegungsschrift US 2013/0018493 A1 zeigt ein Ballabgabesystem für Tennisbälle. Der Schutzumfang der Erfindung wird durch die Ansprüche bestimmt. Die folgenden Ausführungsformen definieren nur Beispiele, nur diejenigen, die in den Schutzbereich der Ansprüche fallen, sind Teil der Erfindung.

Moderne Trainingskonzepte stützen sich oft auf Training unterstützende Geräte, wie etwa Ballabgabevorrichtungen zur Abgabe von Bällen in Richtung eines Spielers. Derartige Ballabgabevorrichtungen werden insbesondere zur Intensivierung eines Fußball- oder Tennisspieltrainings eingesetzt, denn durch die sequenzielle Abgabe von Bällen kann ein Spieler innerhalb einer kurzen Zeitspanne gezielt technische Fertigkeiten wie beispielsweise die Ballannahme trainieren.

Mit den bekannten Ballabgabevorrichtungen ist es jedoch nicht möglich, neben einem Training mit dem Ball auch ein gezieltes individuelles kontrolliertes konditionelles Training zu absolvieren.

Es ist daher die Aufgabe der vorliegenden Erfindung, eine trainingseffizientere Abgabevorrichtung zur Abgabe eines Spielgegenstandes zu schaffen, die es ermöglicht sensordatenbasiert und in Echtzeit konditionelle, technische, taktische und psychologische Trainingsziele synchron und aufeinander abgestimmt individualisiert zu trainieren

Diese Aufgabe wird durch die Merkmale der unabhängigen Ansprüche gelöst. Vorteilhafte Ausführungsformen sind Gegenstand der Beschreibung, der abhängigen Ansprüche sowie der Figuren.

Die vorliegende Erfindung basiert auf der Erkenntnis, dass die obige Aufgabe durch eine Steuerung bzw. Regelung einer Abgabe eines Spielgegenstandes in Abhängigkeit von zumindest einem leistungsphysiologischen Parameter oder mehrerer leistungsphysiologischer Parameter eines Spielers, beispielsweise dessen Herzfrequenz und/oder Laktatwert, gelöst werden kann. Dadurch können gleichzeitig ein konditionelles Training bei beispielsweise einer konstanten oder vorgegebenen Herzfrequenz und ein Fertigkeitstraining zur Verbesserung der Beherrschung des Spielgegenstandes effizient und zeitsparend durchgeführt werden.

Gemäß einem Aspekt betrifft die Erfindung eine Abgabevorrichtung zur Abgabe eines Spielgegenstandes, mit einem Antrieb mit einem Antriebskörper zum Antreiben des Spielgegenstandes gemäß einer regelbaren Antriebscharakteristik, und einer Regelungseinrichtung zum Regeln der regelbaren Antriebscharakteristik in Abhängigkeit von einem leistungsphysiologischen Parameter oder von mehreren leistungsphysiologischen Parametern.

Der Antriebskörper treibt gemäß einer Ausführungsform den Spielgegenstand unmittelbar an. Somit wirkt sich die Antriebscharakteristik unmittelbar auf einen Bewegungszustand des Spielgegenstandes aus.

Die Antriebscharakteristik kann daher eine translatorische und/oder rotatorische Geschwindigkeit des Antriebskörpers und/oder eine translatorische und/oder rotatorische Beschleunigung des Antriebskörpers umfassen. Der Antriebskörper treibt beispielsweise aufgrund einer Wechselwirkung zwischen einer Oberfläche des Antriebskörpers und einer Oberfläche des Spielgegenstandes den Spielgegenstand an.

Durch die regelbare Antriebscharakteristik kann beispielswese eine gewünschte Abgabebahn, beispielsweise eine Wurfbahn, und/oder eine Rotation bzw. ein Spin und/oder eine Abgaberichtung, insbesondere eine vertikale und/oder horizontale Abgaberichtung, und/oder die Abgabestärke des Spielgegenstandes eingestellt werden.

Die Abgabevorrichtung kann ein Sporttrainingsgerät, beispielsweise ein Ballschussroboter, sein, mit welchem ein Spieler mit dem Spielgegenstand trainieren kann. Durch die regelbare Antriebscharakteristik kann besonders effizient eine Trainingsintensität mit dem Spielgegenstand eingestellt bzw. geregelt werden.

Ist der leistungsphysiologische Parameter beispielsweise ein Vitalparameter des Spielers, beispielsweise dessen gemessene Herzfrequenz, so kann die Antriebscharakteristik und somit die Trainingsintensität zudem in Abhängigkeit der gemessenen Herzfrequenz geregelt werden. Auf diese Weise kann der Spieler ein Konditionstraining, ein Regenerationstraining oder ein Aufwärmtraining durchführen und gleichzeitig die Beherrschung des Spielgegenstandes, beispielweise eine Ballannahme, trainieren. Dadurch wird ein besonders zeit-effizientes Training erreicht.

Die Abgabevorrichtung kann einen Spielgegenstandspeicher umfassen, um dem Antrieb einen oder mehrere Spielgegenstände sequenziell zuzuführen.

Gemäß einer Ausführungsform ist die Regelungseinrichtung ausgebildet, die regelbare Antriebscharakteristik in Abhängigkeit von zumindest einem der folgenden leistungsphysiologischen Parametern zu regeln: Vitalparameter, insbesondere Herzfrequenz, Atemfrequenz, Sauerstoffkonzentration, Blutzuckerwert, Blutdruck, Hautleitwiderstand, myoelektrische Aktivität, gehirnelektrische Aktivität, und/oder biomechanischen Parameter, insbesondere einem Zeitparameter, einem biokinematischen Parameter oder einem biodynamischen Parameter.

Gemäß einer Ausführungsform umfasst der leistungsphysiologische Parameter einen oder mehrere Vitalparameter, insbesondere Herzfrequenz, Atemfrequenz, Sauerstoffkonzentration, Blutzuckerwert, Blutdruck, Hautleitwiderstand, myoelektrische Aktivität, gehirnelektrische Aktivität, und/oder mehrere oder ein biomechanisches Merkmal, insbesondere ein Zeitmerkmal, ein biokinematisches Merkmal oder ein biodynamisches Merkmal, und/oder mehrere oder einen biomechanischen Parameter, insbesondere einen Zeitparameter, einen biokinematischen Parameter oder einen biodynamischen Parameter.

Gemäß einer Ausführungsform umfasst der leistungsphysiologische Parameter einen leistungsphysiologischen Soll-Parameter und/oder einen leistungsphysiologischen Ist-Parameter, insbesondere eines Spielers.

Der leistungsphysiologische Soll-Parameter kann ein Soll-Vitalparameter, beispielsweise eine Soll-Herzfrequenz eines Spielers, oder ein biomechanischer Soll-Parameter, beispielsweise eine Soll-Beschleunigung eines Spielers, sein. Der Soll-Parameter kann beispielsweise in einem Speicher abgelegt sein und beispielsweise eine Trainingsvorgabe umsetzen. Der leistungsphysiologische Ist-Parameter ist beispielsweise ein aktueller leistungsphysiologischer Parameter des Spielers, beispielsweise die aktuelle Herzfrequenz oder die Laufgeschwindigkeit.

Der leistungsphysiologische Parameter kann messtechnisch, beispielsweise mittels eines Sensors oder mehrerer Sensoren am Spieler, erfasst werden. Gemäß einer Ausführungsform wird der erfasste leistungsphysiologische Parameter zu der Abgabevorrichtung beispielsweise drahtlos oder drahtgebunden übertragen und von einer Kommunikationsschnittstelle der Abgabevorrichtung empfangen. Der leistungsphysiologische Parameter kann jedoch gemäß einer Ausführungsform mittels einer Eingabeeinrichtung, beispielsweise einer Tastatur oder eines Parameterauswahlfeldes, in ein Eingabefeld der Abgabeeinrichtung eingegeben oder in einem Parameterauswahlfeld ausgewählt werden.

Gemäß einer Ausführungsform umfasst die Abgabevorrichtung eine Kommunikationsschnittstelle, welche ausgebildet ist, den leistungsphysiologischen Parameter oder eine Information über den Parameter, insbesondere zumindest einen Messwert des physiologischen Parameters, über ein Kommunikationsnetzwerk zu empfangen. Der leistungsphysiologische Parameter kann ein leistungsphysiologischer Ist-Parameter eines Spielers, beispielsweise ein gemessener leistungsphysiologischer Ist-Parameter eines Spielers, sein.

Gemäß einer Ausführungsform ist der leistungsphysiologische Parameter ein Ist-Parameter oder umfasst der leistungsphysiologische Parameter einen Ist-Parameter eines Spielers, wobei die Regelungseinrichtung ausgebildet ist, die regelbare Antriebscharakteristik des Antriebs in Abhängigkeit des leistungsphysiologischen Ist-Parameters und eines leistungsphysiologischen Soll-Parameters, insbesondere in Abhängigkeit von einem Unterschied bzw. Differenz zwischen dem leistungsphysiologischen Ist-Parameter und dem leistungsphysiologischen Soll-Parameter, zu regeln. Die Regelungseinrichtung kann beispielsweise eine rückgekoppelte Regelstruktur aufweisen und die Antriebscharakteristik auf der Basis der Differenz zwischen dem leistungsphysiologischen Ist-Parameter und Soll-Parameter zu steuern. Die Regelungseinrichtung kann hierbei ausgebildet sein, die Antriebscharakteristik derart zu steuern, sodass die Differenz minimiert wird.

Gemäß einer Ausführungsform ist der leistungsphysiologische Parameter ein Soll- Parameter, wobei die regelbare Regelungseinrichtung ausgebildet ist, die regelbare Antriebscharakteristik auf der Basis, insbesondere nur auf der Basis, des leistungsphysiologischen Soll-Parameters zu regeln. Hierbei kann die Regeleinrichtung anhand eines in einem Speicher vorgespeicherten Zusammenhanges, beispielsweise einer Kennlinie, zwischen leistungsphysiologischen Soll-Parametern und Antriebscharakteristika die Antriebscharakteristik bestimmen bzw. auslesen und den Antrieb entsprechend der ausgelesenen Antriebscharakteristik regeln bzw. einstellen.

Gemäß einer Ausführungsform umfasst die Abgabevorrichtung einen Speicher, beispielsweise den vorgenannten Speicher, zum Speichern einer Mehrzahl von auswählbaren leistungsphysiologischen Parametern, insbesondere einer Mehrzahl von leistungsphysiologischen Soll- Parametern, wobei der leistungsphysiologische Parameter, insbesondere als ein leistungsphysiologischer Soll-Parameter mittels einer graphischen Benutzerschnittstelle auswählbar ist. Mit dem ausgewählten leistungsphysiologischen Soll-Parameter kann die Regeleinrichtung die Antriebscharakteristik unter Verwendung eines vorgespeicherten Zusammenhanges zwischen Soll-Parametern und Antriebscharakteristika, beispielsweise einer Kennlinie, einstellen bzw. regeln.

Gemäß einer Ausführungsform ist der Antriebskörper ein transversal verschiebbarer Antriebskörper, wobei die Antriebscharakteristik eine lineare Antriebscharakteristik ist, oder wobei der Antriebskörper ein rotierbarer Antriebskörper ist und die Antriebscharakteristik eine Rotationscharakteristik ist, oder wobei der Antriebskörper ein rotierbarer Antriebskörper ist und wobei der Antrieb ferner einen transversal verschiebbareren Antriebskörper umfasst, und wobei die Antriebscharakteristik eine lineare Antriebscharakteristik des transversal verschiebbareren Antriebskörpers und eine Rotationscharakteristik des rotierbaren Antriebskörpers umfasst. Der transversal verschiebbare Antriebskörper kann beispielsweise eine bewegliche Antriebsstange mit einer Stirnseite zur Übertragung eines Stoßimpulses auf den Spielgegenstand umfassen.

Gemäß einer Ausführungsform ist der Antriebskörper ein rotierbarer Antriebskörper, wobei die Regelungseinrichtung ausgebildet ist, zur Regelung der regelbaren Antriebscharakteristik zumindest einen der folgenden Rotationsparameter des rotierbaren Antriebskörpers zu regeln: Rotationsgeschwindigkeit, Rotationsrichtung, Rotationsbeschleunigung, Rotationsdauer, Neigung einer Rotationsachse, Neigung einer Rotationsachse bezüglich eines senkrechten Lots, Neigung des Antriebskörpers, insbesondere einer Flächennormalen, bezüglich eines senkrechten Lots bzw. einer Waagerechten.

Gemäß einer Ausführungsform ist der Antriebskörper ein rotierbarer Antriebskörper, wobei der Antrieb einen zweiten rotierbaren Antriebskörper umfasst, und wobei der Spielgegenstand mittels des rotierbaren Antriebskörpers und des zweiten rotierbaren Antriebskörpers antreibbar ist. Beide Antriebskörper können hierbei in entgegengesetzte Richtungen rotieren, sodass der zwischen den Antriebskörpern eingebrachte Spielgegenstand von beiden Antriebskörpern angetrieben und abgegeben wird.

Gemäß einer Ausführungsform umfasst der zweite rotierbare Antriebskörper eine zweite regelbare Antriebscharakteristik, insbesondere eine Rotationscharakteristik, wobei die Regelungseinrichtung ausgebildet ist, die zweite Antriebscharakteristik in Abhängigkeit von dem leistungsphysiologischen Parameter zu regeln. Die zweite Antriebscharakteristik kann dieselben Merkmale wie die erste Antriebscharakteristik umfassen. Gemäß einer Ausführungsform ist die Regelungseinrichtung ausgebildet, beide Antriebskörper gleichzeitig zu regeln. Bei gleicher Rotationsgeschwindigkeit und unterschiedlichen Rotationsrichtungen der Antriebskörper wird der Spielgegenstand beispielsweise weniger (oder gar nicht) in Eigenrotation versetzt als bei einem Unterschied der Rotationsgeschwindigkeiten der Antriebskörper.

Gemäß einer Ausführungsform umfasst der Antrieb eine Wandung, an welcher der Spielgegenstand führbar oder abrollbar ist, und wobei der Spielgegenstand zwischen den Antriebskörper und die Wandung einbringbar ist. Der Spielgegenstand kann daher, angetrieben durch den Antriebskörper, an der Wandung abrollen und abgegeben werden.

Gemäß einer Ausführungsform ist oder umfasst der jeweilige Antriebskörper eine Antriebsscheibe oder eine Antriebskugel oder einen Antriebskegel. Durch eine Rotation des Antriebskörpers kann der Spielgegenstand in Eigenrotation versetzt und abgegeben bzw. freigegeben werden.

Gemäß einer Ausführungsform ist der jeweilige Antriebskörper drehbar, insbesondere um eine außerhalb des jeweiligen Antriebskörpers liegende Drehachse drehbar, gelagert wobei die Regelungseinrichtung ausgebildet ist, den Antriebskörper in Abhängigkeit von dem Parameter oder in Abhängigkeit von einer Abgaberichtung, insbesondere von einer zufällig auswählbaren oder einer einstellbaren Abgaberichtung, zu drehen. Auf diese Weise kann eine Raumlage des jeweiligen Antriebskörpers geändert werden, um beispielsweise den Spielgegenstand in unterschiedliche Richtungen abgeben zu können. Die Drehachse kann eine beliebige Raumachse sein.

Gemäß einer Ausführungsform ist der Antriebskörper ein transversal verschiebbarer Antriebskörper, welcher ausgebildet ist, einen Stoßimpuls auf den Spielgegenstand auszuüben. In diesem Fall kann der Spielgegenstand durch einen linearen Stoß angetrieben und abgegeben werden. Dabei bestimmen eine Stoßrichtung, eine Stoßstärke bzw. ein Stoßversatz bezüglich eines Schwerpunktes des Spielgegenstandes die Antriebscharakteristik und somit eine Abgabebahn des Spielgegenstandes.

Gemäß einer Ausführungsform ist der Antriebskörper ein transversal verschiebbarer Antriebskörper, dessen lateraler Versatz und/oder Winkelversatz einstellbar ist. Durch den lateralen Versatz und durch den Winkelversatz wird eine Stoßrichtung bezüglich einer waagerecht verlaufenden bzw. zur Lotrichtung senkrecht verlaufenden Achse durch einen Schwerpunkt des Spielgegenstandes bestimmt. Auf diese Weise kann ein dezentraler Stoßimpuls den Spielgegenstand in Eigenrotation versetzen und/oder entlang einer Wurfparabel befördern.

Gemäß einer Ausführungsform ist der Antriebskörper ein transversal verschiebbarer Antriebskörper, wobei die Antriebcharakteristik eine lineare Antriebscharakteristik ist, und wobei die Regelungseinrichtung ausgebildet ist, zur Regelung der regelbaren Antriebscharakteristik zumindest einen der folgenden Parameter des Antriebskörpers zu regeln: Impuls, Beschleunigung, lateralen Versatz bezüglich des Spielgegenstandes, Winkelversatz bezüglich des Spielgegenstandes, oder Neigung des Antriebskörpers bezüglich eines senkrechten Lots.

Gemäß einer Ausführungsform ist die Abgabevorrichtung ausgebildet, eine Mehrzahl von Spielgegenständen in einem vorbestimmten, insbesondere einstellbaren, Zeitabstand abzugeben. Der Zeitabstand kann beispielsweise ebenfalls von dem leistungsphysiologischen Parameter abhängen. So kann der Zeitabstand beispielsweise zur Erhöhung einer Herzfrequenz eines Spielers verkürzt oder zur Verringerung einer Herzfrequenz eines Spielers verlängert werden. Durch den Zeitabstand kann eine regelbare Abgabefrequenz des Spielgegenstandes eingestellt werden. Der Zeitabstand kann beispielsweise durch die Regelungseinrichtung gesteuert werden. Hierzu kann der Antrieb aktiviert oder deaktiviert werden und/oder die Spielgegenstände können dem Antrieb gemäß dem Zeitabstand zugeführt werden. Die Abgabevorrichtung kann einen Spielgegenstandspeicher umfassen, um dem Antrieb jeweils einen Spielgegenstand zuzuführen.

Gemäß einer Ausführungsform ist die Abgabevorrichtung ausgebildet, vor oder mit der Abgabe des Spielgegenstandes ein Abgabehinweissignal, insbesondere ein akustisches oder optisches Abgabehinweissignal, zu erzeugen. Auf diese Weise kann der Spieler in Kenntnis der bevorstehenden Abgabe des Spielgegenstandes gesetzt werden. Das Zeitintervall zwischen der Erzeugung des Abgabehinweissignals und der Abgabe des Spielgegenstandes kann beispielsweise in Abhängigkeit von dem leistungsphysiologischen Parameter, beispielsweise durch die Regelungseinrichtung, einstellbar sein. Auf diese Weise kann in Echtzeit ein Training mit dem Spielgegenstand individuell und adaptiv durchgeführt werden.

Gemäß einer Ausführungsform ist der Spielgegenstand ein Ball, insbesondere ein Fußball, ein American Football, ein Rugby Ball, ein Basketball, ein Baseball, ein Volleyball, ein Handball, ein Golfball, ein Cricket-Ball, ein Polo-Ball, ein Tischtennisball, oder wobei der Spielgegenstand ein Hockey-Puck ist.

Gemäß einem Aspekt betrifft die Erfindung ein Verfahren zur Abgabe eines Spielgegenstandes mittels eines Antriebkörper, welcher einen rotierbaren Antriebskörper zum Antreiben des Spielgegenstandes gemäß einer regelbaren Antriebscharakteristik umfasst, mit: Regeln der regelbaren Antriebscharakteristik in Abhängigkeit von zumindest einem leistungsphysiologischen Parameter.

Weitere Merkmale des Verfahrens zur Abgabe eines Spielgegenstandes ergeben sich unmittelbar aus Merkmalen der Vorrichtung zur Abgabe eines Spielgegenstandes.

Gemäß einer Ausführungsform wird das Verfahren durch die Abgabevorrichtung zur Abgabe eines Spielgegenstandes ausgeführt.

Gemäß einem Aspekt betrifft die Erfindung ein Trainingssystem, mit einer Messeinrichtung zur Erfassung zumindest eines leistungsphysiologischen Parameters eines Spielers und der erfindungsgemäßen Abgabevorrichtung zur Abgabe eines Spielgegenstandes in Abhängigkeit von dem leistungsphysiologischen Parameter des Spielers.

Die Messeinrichtung kann einen Sensor oder mehrere Sensoren umfassen, um den leistungsphysiologischen Parameter, beispielswiese einen Vitalparameter oder einen biomechanischen Parameter, oder um mehrere leistungsphysiologische Parameter zu erfassen. Der jeweils erfasste leistungsphysiologische Parameter kann beispielsweise als der vorgenannte leistungsphysiologische Ist-Parameter zur Regelung der Antriebscharakteristik durch die Regelungseinrichtung eingesetzt werden.

Weitere Ausführungsbeispiele werden Bezug nehmend auf die beiliegenden Figuren erläutert. Es zeigen:
- Fig. 1: eine schematische Darstellung einer Abgabevorrichtung;
- Fig. 2: eine schematische Darstellung einer Abgabevorrichtung; und
- Fig. 3: eine schematische Darstellung eines Trainingssystems.

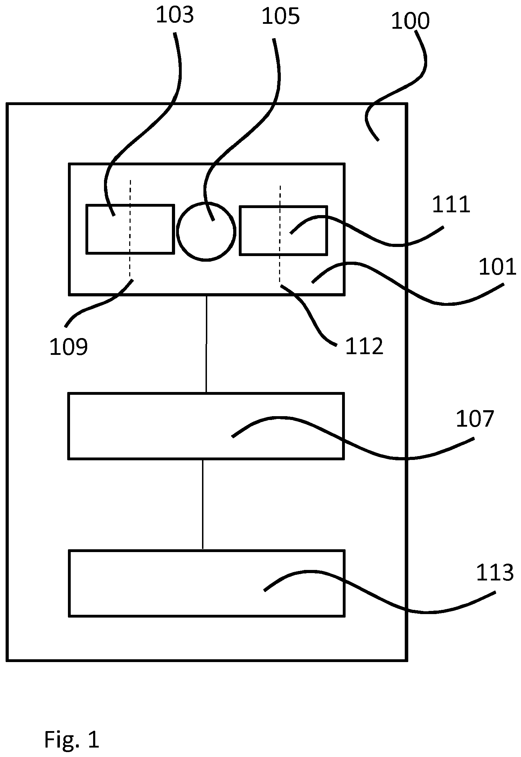

Fig. 1 zeigt eine Abgabevorrichtung 100 zur Abgabe eines Spielgegenstandes mit einem Antrieb 101 mit einem Antriebskörper 103 zum Antreiben des Spielgegenstandes 105 gemäß einer regelbaren Antriebscharakteristik, und einer Regelungseinrichtung 107 zum Regeln der regelbaren Antriebscharakteristik in Abhängigkeit von zumindest einem leistungsphysiologischen Parameter oder mehreren leistungsphysiologischen Parametern.

Der Antriebskörper 103 kann beispielsweise ein um die Achse 109 rotierbarer Antriebskörper, beispielsweise eine Kugel, eine Scheibe oder ein Kegel, sein. Durch die Rotation des Antriebskörpers 103 wird der Spielgegenstand 105, beispielsweise ein Fußball, in Rotation versetzt und beispielsweise in Richtung eines in Fig. 1 nicht gezeigten Spielers, abgegeben.

Die Abgabevorrichtung 100 kann ferner ein Antriebselement 111 umfassen, das mit dem Spielgegenstand 105 ebenfalls wechselwirken kann.

Gemäß einer Ausführungsform ist das Antriebselement 111 ein zweiter rotierbarer Antriebskörper 111, dessen Rotationsrichtung um die Rotationsachse 112 entgegengesetzt zu der Rotationsrichtung des (ersten) rotierbaren Antriebskörpers 103 ist. Der zweite rotierbare Antriebskörper 111 kann die Merkmale des ersten rotierbaren Antriebskörpers aufweisen. Durch entgegen gesetzte Rotationen der rotierbaren Antriebskörper 103, 111 kann der dazwischen anordenbare Spielgegenstand beschleunigt und abgegeben werden. Die Rotationsgeschwindigkeiten der rotierbaren Antriebskörper 103, 111 können gleich oder unterschiedlich sein, um beispielsweise zusätzlich eine Eigenrotation des Spielgegenstandes und/oder dessen Abgaberichtung zu beeinflussen.

Gemäß einer weiteren Ausführungsform ist das Antriebselement 111 eine Wandung, an welcher der Spielgegenstand 105, angetrieben durch den rotierbaren Antriebskörper 103, abrollen kann. Auf diese Weise wird der Spielgegenstand beispielsweise entlang der Wandung 111 geführt, deren Ausrichtung im Raum eine Abgaberichtung des Spielgegenstandes beeinflussen kann.

Der Antrieb 101 kann zum Antreiben des jeweiligen Antriebsköpers103, 111 einen steuerbaren oder mehrere steuerbare Motoren umfassen, um die jeweilige Antriebscharakteristik wie beispielsweise Rotationsbeschleunigung, Rotationsgeschwindigkeit oder Rotationsrichtung zu steuern bzw. zu regeln.

Die Regelung kann beispielsweise darin bestehen, die Antriebscharakteristik in Abhängigkeit von einer Differenz zwischen einem oder mehreren leistungsphysiologischen Ist-Parameter, beispielsweise einer Herzfrequenz des Spielers, und einem oder mehreren leistungsphysiologischen Soll-Parameter derart zu regeln bzw. zu steuern, dass die Differenz minimiert wird.

Die Regelung kann jedoch auch ausschließlich auf der Basis eines leistungsphysiologischen Soll-Parameters erfolgen. Hierzu kann der Soll-Parameter beispielsweise mittels einer graphischen Eingabeeinrichtung eingebbar sein.

Die Regelung kann adaptiv und/oder auf der Basis von in einem Speicher der Abgabevorrichtung 100 abgespeicherten Kennlinien erfolgen, welche einen Zusammenhang zwischen leistungsphysiologischen Parametern, insbesondere leistungsphysiologischen Ist-Parametern, bzw. einer Differenz zwischen Ist- und Soll-Parametern und Antriebscharakteristika, festlegen.

Die Abgabevorrichtung 100 umfasst gemäß einer Ausführungsform eine Kommunikationsschnittstelle 113, welche für eine drahtlose Kommunikation gemäß einem drahtlosen Kommunikationsstandard oder für eine drahtgebundene Kommunikation gemäß einem drahtgebundenen Kommunikationsstandard ausgelegt sein kann. Mittels der Kommunikationsschnittstelle kann die Abgabevorrichtung 100 beispielsweise einen oder mehrere leistungsphysiologischen Parameter empfangen. Die Kommunikationsschnittstelle 113 ist mit der Regelungseinrichtung 107 verbunden und übermittelt an diese den empfangenen leistungsphysiologischen (Ist-)Parameter zur Regelung der Antriebscharakteristik.

Der oder die empfangbaren leistungsphysiologischen Parameter können beispielsweise ein leistungsphysiologischer Ist-Parameter des Spielers sein, welcher von einer Messeinrichtung, welche an dem in Fig. 1 nicht dargestellten Spieler angebracht sein kann, beispielsweiser ein Pulssensor, ausgesendet und durch die Kommunikationsschnittstelle 113 empfangen wird.

Die Kommunikationsschnittstelle 115 kann gemäß einer Ausführungsform zur Fernbedienung der Abgabevorrichtung 100 oder zur Übermittlung bzw. Einstellung eines oder mehrerer leistungsphysiologischer Soll-Parameter dienen.

Gemäß einer Ausführungsform umfasst die Abgabevorrichtung 100 ferner einen transversal verschiebbaren Antriebskörper, welcher einen geführten Stoß auf den Spielgegenstand ausüben kann. Hierbei umfasst die Antriebscharakteristik auch eine lineare Antriebscharakteristik des transversal verschiebbareren Antriebskörpers, welche durch die Regelungseinrichtung 107 eingestellt wird. Zum Antreiben des transversal verschiebbareren Antriebskörpers kann der Antrieb zusätzlich einen Linearantrieb oder ein Getriebe zur Umsetzung einer Rotation in eine Linearbewegung umfassen. Durch eine Kombination der Rotationscharakteristik des rotierbaren Antriebskörpers 103, 111 und der linearen Antriebscharakteristik kann eine Abgabebahn bzw. eine Abgabetrajektorie des Spielgegenstandes und/oder dessen Eigenbewegung, wie etwa Eigenrotation um die eigene Rotationsachse, eingestellt werden.

Gemäß einer Ausführungsform umfasst die Abgabevorrichtung 100 nur den transversal verschiebbareren Antriebskörper.

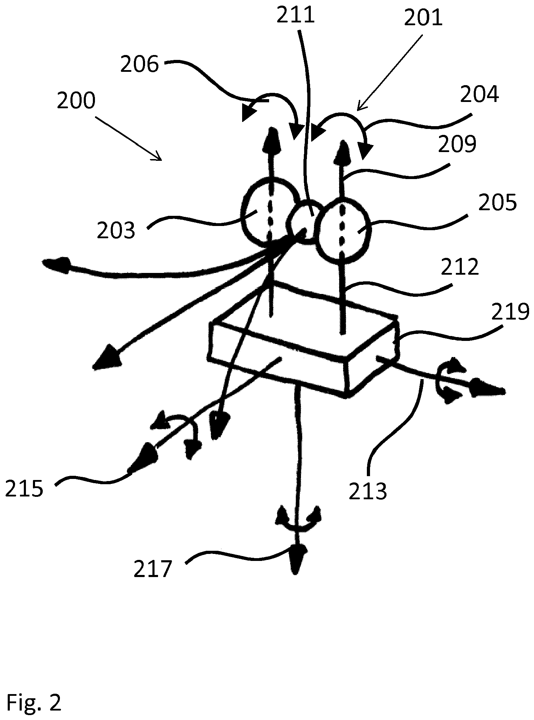

Gemäß einer Ausführungsform kann der Antrieb drehbar gelagert sein, wobei der jeweilige Antriebskörper 103, 111, zusätzlich um zumindest eine weitere Drehachse drehbar ist. Dadurch kann die Abgabe des Spielgegenstandes in eine beliebige Raumrichtung erfolgen. Diese Ausführungsform ist in Fig. 2 dargestellt.

Fig. 2 zeigt schematisch eine Abgabevorrichtung 200 mit einem Antrieb 201, welcher beispielhaft zwei rotierbare Antriebskörper 203 und 205 umfasst. Die rotierbaren Antriebskörper 203 und 205 sind um deren Rotationsachsen 207 und 209 wie in Fig. 2 durch die Pfeile 204 und 206 angedeutet jeweils beispielswiese in beide Richtungen rotierbar gelagert. Dadurch kann beispielsweise ein Spin eines Fußballs, eingestellt werden.

Eine in Fig. 2 nicht dargestellte Regelungseinrichtung kann die Antriebscharakteristika der rotierbaren Antriebskörper 203 und 205 wie im Zusammenhang mit dem in Fig. 1 dargestellten Ausführungsbeispiel beschrieben regeln bzw. steuern. Dadurch kann die Abgabe eines zwischen die rotierbaren Antriebskörper einbringbaren Spielgegenstandes 211, beispielsweise eines Fußballs, bewirkt werden.

Die Abgabevorrichtung 200 kann mit den rotierbaren Antriebskörpern 203 und 205 sowie der nicht dargestellten Regelungseinrichtung und einer optionalen Kommunikationsschnittstelle die Merkmale der Abgabevorrichtung 200 umfassen.

Darüber hinaus können die Antriebskörper 203 und 205 gemeinsam um eine oder mehrere der Drehachsen 213, 215 und 217 im Raum drehbar gelagert sein. Die Drehachsen 213, 215 und 217 liegen beispielsweise außerhalb der Antriebskörper 203 und 205 und bewirken eine Ausrichtung der Antriebskörper 203 und 205 im Raum, um unterschiedliche Trajektorien des abgegebenen Spielgegenstandes 211 zu erreichen. Hierzu kann ein weiterer Antrieb 219 vorgesehen sein, um die Antriebskörper 203 und 205 gemeinsam im Raum zu schwenken, zu neigen oder zu verdrehen. Der weitere Antrieb 219 kann beispielsweise einen oder mehrere Motoren umfassen, welche zur Einstellung eines Raumwinkels eine Drehung der Antriebskörper 203 und 205 um eine oder mehrere der Drehachsen 213, 215, 217 bewirkt oder bewirken. Der weitere Antrieb 219 kann durch die Regelungseinrichtung steuerbar bzw. regelbar sein.

Gemäß einer Ausführungsform kann einer der Antriebskörper 203, 205 durch eine Wandung zum Abrollen des Spielgegenstandes 211 ersetzen.

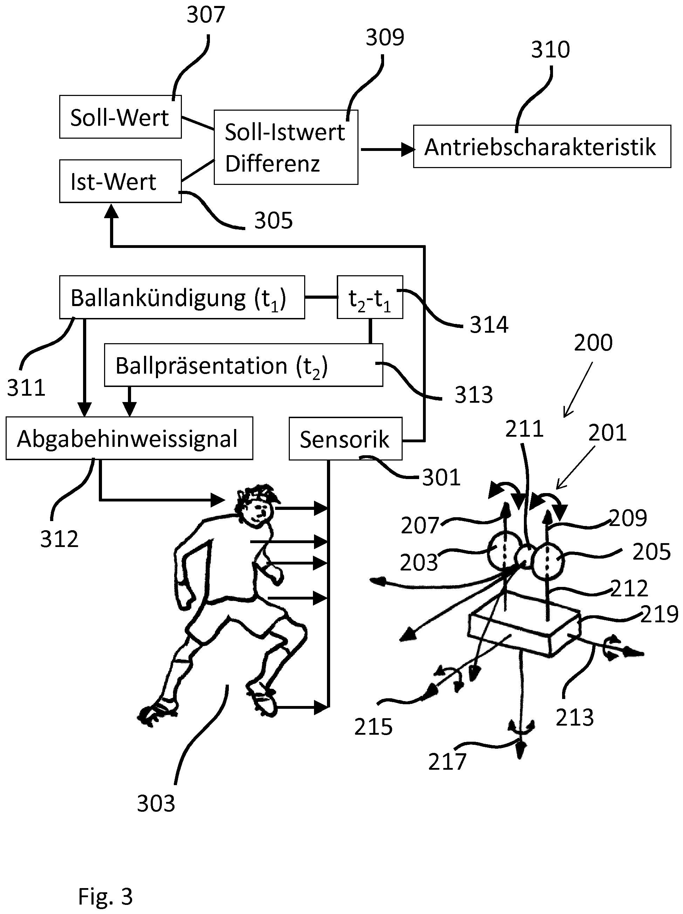

Fig. 3 zeigt ein Trainingssystem, mit einer Messeinrichtung 301 zur Erfassung zumindest eines leistungsphysiologischen Parameters eines Spielers 303 und beispielsweise der Abgabevorrichtung 200 zur Abgabe des Spielgegenstandes 211 in Abhängigkeit von dem leistungsphysiologischen Parameter des Spielers 303. Die nachfolgenden Ausführungen gelten jedoch analog auch für die Verwendung der in Fig. 1 dargestellten Abgabevorrichtung 100 in dem in Fig. 3 dargestellten Trainingssystem.

Nachfolgend wird exemplarisch ein Ablauf eines Trainingsverfahrens unter Verwendung des Trainingssystems beschrieben.

Die Messeinrichtung 301 umfasst beispielsweise einen oder mehrere Sensoren, welche am Spieler 303 angeordnet sein können. Die Sensoren erfassen den oder die aktuellen leistungsphysiologischen Ist-Parameter des Spielers 303, beispielsweise seine Herzfrequenz, seine Atemfrequenz, seine Laufgeschwindigkeit oder Laktatwerte.

Der oder die leistungsphysiologischen Ist-Parameter des Spielers 303 werden durch die Messeinrichtung 301 im Schritt 305 an die Abgabevorrichtung 200 beispielsweise über ein drahtloses Kommunikationsnetzwerk durch die Verwendung einer drahtlosen Kommunikationstechnologie wie Bluetooth, WLAN oder Infrarot übermittelt.

Der oder die leistungsphysiologischen Ist-Parameter werden durch eine in Fig. 3 nicht dargestellte Kommunikationsschnittstelle empfangen. Im Schritt 307 wird oder werden leistungsphysiologische Soll-Parameter beispielsweise aus einem Speicher der Abgabevorrichtung 200 durch beispielsweise die Regelungseinrichtung ausgelesen. Der oder die leistungsphysiologischen Soll-Parameter werden im Schritt 309 mit zumindest einem der empfangenen leistungsphysiologischen Ist-Parametern verglichen, um eine Soll-Istwert-Differenz zu erhalten. Auf der Basis der Soll-Istwert-Differenz regelt oder steuert die Regelungseinrichtung im Schritt 310 die jeweilige Antriebscharakteristik des jeweiligen Antriebskörpers, um beispielswiese im Falle eines Balls eine Ballabgabefrequenz, einen Ballspin, eine Balltrajektorie oder eine Ballgeschwindigkeit zu regeln.

Die bevorstehende Abgabe des Spielgegenstandes 211 kann durch die Abgabevorrichtung 200 mittels eines akustischen, optischen oder akusto-optischen Abgabehinweissignals 312 zum Zeitpunkt t1 im Schritt 311 angezeigt werden. Die Abgabe des Spielgegenstandes erfolgt im Schritt 313 zum Zeitpunkt t2 nach Ablauf 314 einer Zeitdifferenz t2-t1. Die Zeitdifferenz t2-t1 kann durch die Regelungseinrichtung ebenfalls in Abhängigkeit zumindest eines leistungsphysiologischen Ist-Parameters eingestellt werden, um beispielsweise eine Trainingsintensität zu erhöhen oder zu verringern.

Das vorstehende beschriebene Verfahren kann gemäß einer Ausführungsform adaptiv ausgeführt werden. Hierbei werden durch die Abgabevorrichtung 200 mehrere Spielgegenstände nacheinander abgegeben, und der oder die leistungsphysiologischen Ist-Parameter des Spielers 303 werden kontinuierlich überwacht und an die Abgabevorrichtung 200 übertragen.

Gemäß einer Ausführungsform wird die Regelung der Antriebscharakteristik des jeweiligen Antriebskörpers 203, 205 nur dann durchgeführt, wenn die Soll-Istwert-Differenz einen vorbestimmten oder einstellbaren Schwellwert erreicht oder überschreitet. Der Schwellwert kann beispielsweise 2%, 5%, 10%, 15% oder 20%, bezogen auf einen Ist-Wert oder Soll-Wert, betragen.

Die beschriebenen Ausführungsbeispiele beziehen sich lediglich beispielhaft auf einen leistungsphysiologischer Parameter bzw. einen leistungsphysiologischer Ist-Parameter oder einen leistungsphysiologischer Soll-Parameter. In sämtlichen Ausführungsbeispielen können jedoch mehrere leistungsphysiologische Parameter bzw. leistungsphysiologische Ist-Parameter oder leistungsphysiologische Soll-Parameter analog verwendet werden.

## Patentansprüche

1. Abgabevorrichtung (100, 200) zur Abgabe eines Spielgegenstandes, mit:
einem Antrieb (101, 201) mit einem Antriebskörper (103, 111, 203, 205) zum Antreiben des Spielgegenstandes gemäß einer regelbaren Antriebscharakteristik; und
einer Regelungseinrichtung (107) zum Regeln der regelbaren Antriebscharakteristik in Abhängigkeit von zumindest einem leistungsphysiologischen Parameter,
wobei der leistungsphysiologische Parameter ein Ist-Parameter eines Spielers ist, wobei die Regelungseinrichtung ausgebildet ist, die regelbare Antriebscharakteristik des Antriebs in Abhängigkeit des leistungsphysiologischen Ist-Parameters und eines leistungsphysiologischen Soll-Parameters zu regeln, wobei die Regelung der Antriebscharakteristik des Antriebskörpers nur dann durchgeführt wird, wenn die Soll-Ist-Wert-Differenz einen vorbestimmten oder einstellbaren Schwellwert erreicht oder überschreitet.

2. Abgabevorrichtung nach Anspruch 1, wobei die Regelungseinrichtung (107) ausgebildet ist, die regelbare Antriebscharakteristik in Abhängigkeit von zumindest einem der folgenden leistungsphysiologischen Parametern zu regeln: Vitalparameter, insbesondere Herzfrequenz, Atemfrequenz, Sauerstoffkonzentration, Blutzuckerwert, Blutdruck, Hautleitwiderstand, myoelektrische Aktivität, gehirnelektrische Aktivität, oder biomechanischen Parameter, insbesondere einem Zeitparameter, einem biokinematischen Parameter oder einem biodynamischen Parameter.

3. Abgabevorrichtung nach einem der vorstehenden Ansprüche, wobei der leistungsphysiologische Parameter einen leistungsphysiologischen Soll-Parameter oder einen leistungsphysiologischen Ist-Parameter, insbesondere einen leistungsphysiologischen Soll-Parameter oder einen leistungsphysiologischen Ist-Parameter eines Spielers, umfasst.

4. Abgabevorrichtung nach einem der vorstehenden Ansprüche, mit einer Kommunikationsschnittstelle (113), welche ausgebildet ist, den leistungsphysiologischen Parameter oder eine Information über den leistungsphysiologischen Parameter, insbesondere zumindest einen Messwert des leistungsphysiologischen Parameters, über ein Kommunikationsnetzwerk zu empfangen.

5. Abgabevorrichtung nach einem der vorstehenden Ansprüche, mit einem Speicher zum Speichern einer Mehrzahl von auswählbaren leistungsphysiologischen Parametern, insbesondere einer Mehrzahl von Soll-Parametern, wobei der leistungsphysiologische Parameter, insbesondere als ein leistungsphysiologischer Soll-Parameter, mittels einer graphischen Benutzerschnittstelle auswählbar ist.

6. Abgabevorrichtung nach einem der vorstehenden Ansprüche, wobei der Antriebskörper ein transversal verschiebbarer Antriebskörper ist und wobei die Antriebscharakteristik eine lineare Antriebscharakteristik ist, oder wobei der Antriebskörper (103, 111, 203, 205) ein rotierbarer Antriebskörper ist und wobei die Antriebscharakteristik eine Rotationscharakteristik ist, oder wobei der Antriebskörper (103, 111, 203, 205) ein rotierbarer Antriebskörper ist und wobei der Antrieb ferner einen transversal verschiebbareren Antriebskörper umfasst, und wobei die Antriebscharakteristik eine lineare Antriebscharakteristik des transversal verschiebbareren Antriebskörpers und eine Rotationscharakteristik des rotierbaren Antriebskörpers (103, 111, 203, 205) umfasst.

7. Abgabevorrichtung nach einem der vorstehenden Ansprüche, wobei der Antriebskörper (103, 111, 203, 205) ein rotierbarer Antriebskörper (103, 111, 203, 205) ist, und wobei die Regelungseinrichtung (107) ausgebildet ist, zur Regelung der regelbaren Antriebscharakteristik zumindest einen der folgenden Rotationsparameter des Antriebskörpers (103, 111, 203, 205) zu regeln: Rotationsgeschwindigkeit, Rotationsrichtung, Rotationsbeschleunigung, Rotationsdauer, Neigung einer Rotationsachse, Neigung einer Rotationsachse bezüglich eines senkrechten Lots, Neigung des Antriebskörpers.

8. Abgabevorrichtung nach einem der vorstehenden Ansprüche, wobei der Antriebskörper (103, 111, 203, 205) ein rotierbarer (103, 111, 203, 205) Antriebskörper ist, wobei der Antrieb (101, 201) einen zweiten rotierbaren Antriebskörper (103, 111, 203, 205) umfasst, und wobei der Spielgegenstand mittels des rotierbaren Antriebskörpers und des zweiten rotierbaren Antriebskörpers (103, 111, 203, 205) antreibbar ist.

9. Abgabevorrichtung nach Anspruch 8, wobei der zweite rotierbare Antriebskörper (103, 111, 203, 205) eine zweite regelbare Antriebscharakteristik, insbesondere eine Rotationscharakteristik, aufweist, und wobei die Regelungseinrichtung (107) ausgebildet ist, die zweite Antriebscharakteristik in Abhängigkeit von dem leistungsphysiologischen Parameter zu regeln.

10. Abgabevorrichtung nach einem der vorstehenden Ansprüche, wobei der Antrieb (101, 201) eine Wandung umfasst, an welcher der Spielgegenstand führbar oder abrollbar ist, und wobei der Spielgegenstand zwischen den Antriebskörper (103, 111, 203, 205) und die Wandung einbringbar ist.

11. Abgabevorrichtung nach einem der vorstehenden Ansprüche, wobei der jeweilige Antriebskörper (103, 111, 203, 205) eine Antriebsscheibe oder eine Antriebskugel oder einen Antriebskegel umfasst.

12. Abgabevorrichtung nach einem der vorstehenden Ansprüche, wobei der jeweilige Antriebskörper (103, 111, 203, 205) drehbar, insbesondere um eine außerhalb des jeweiligen Antriebskörpers (103, 111, 203, 205) liegende Drehachse drehbar gelagert ist, und wobei die Regelungseinrichtung (107) ausgebildet ist, den Antriebskörper (103, 111, 203, 205) in Abhängigkeit von dem leistungsphysiologischen Parameter oder in Abhängigkeit von einer Abgaberichtung des Spielgegenstandes, insbesondere von einer zufällig auswählbaren oder einer einstellbaren Abgaberichtung, zu drehen.

13. Abgabevorrichtung nach einem der vorstehenden Ansprüche 1 bis 6, wobei der Antriebskörper ein transversal verschiebbarer Antriebskörper ist, welcher ausgebildet ist, einen Stoßimpuls auf den Spielgegenstand auszuüben.

14. Abgabevorrichtung nach Anspruch 13, wobei der Antriebskörper ein transversal verschiebbarer Antriebskörper ist, wobei die Antriebcharakteristik eine lineare Antriebscharakteristik ist, und wobei die Regelungseinrichtung (107) ausgebildet ist, zur Regelung der regelbaren Antriebscharakteristik zumindest einen der folgenden Antriebsparameter des Antriebskörpers zu regeln: Impuls, Beschleunigung, lateralen Versatz bezüglich des Spielgegenstandes, Winkelversatz bezüglich des Spielgegenstandes, oder Neigung des Antriebskörpers bezüglich eines senkrechten Lots.

15. Abgabevorrichtung nach einem der vorstehenden Ansprüche, welche ausgebildet ist, eine Mehrzahl von Spielgegenständen in einem vorbestimmten, insbesondere einstellbaren, Zeitabstand abzugeben.

16. Abgabevorrichtung gemäß einem der vorstehenden Ansprüche, welche ausgebildet ist, vor oder mit der Abgabe des Spielgegenstandes ein Abgabehinweissignal, insbesondere ein akustisches oder optisches Abgabehinweissignal, zu erzeugen.

17. Abgabevorrichtung nach einem der vorstehenden Ansprüche, wobei der Spielgegenstand ein Ball, insbesondere ein Fußball, ein American Football, ein Rugby Ball, ein Basketball, ein Baseball, ein Volleyball, ein Handball, ein Handball, ein Golfball, ein Cricket-Ball, ein Polo-Ball, ein Tischtennisball, ist, oder wobei der Spielgegenstand ein Hockey-Puck ist.

18. Verfahren zur Abgabe eines Spielgegenstandes mittels eines Antriebkörpers, welcher einen rotierbaren Antriebskörper zum Antreiben des Spielgegenstandes gemäß einer regelbaren Antriebscharakteristik umfasst, mit:
Regeln der regelbaren Antriebscharakteristik in Abhängigkeit von zumindest einem leistungsphysiologischen Parameter,
wobei der leistungsphysiologische Parameter ein Ist-Parameter eines Spielers ist, wobei die regelbare Antriebscharakteristik des Antriebs in Abhängigkeit des leistungsphysiologischen Ist-Parameters und eines leistungsphysiologischen Soll-Parameters geregelt wird, wobei die Regelung der Antriebscharakteristik des Antriebskörpers nur dann durchgeführt wird, wenn die Soll-Ist-Wert-Differenz einen vorbestimmten oder einstellbaren Schwellwert erreicht oder überschreitet.

19. Trainingssystem, mit:
einer Messeinrichtung (301) zur Erfassung zumindest eines leistungsphysiologischen Parameters eines Spielers; und
der Abgabevorrichtung (100, 200) zur Abgabe eines Spielgegenstandes gemäß einem der Ansprüche 1 bis 17 in Abhängigkeit von dem leistungsphysiologischen Parameter des Spielers.

## Claims

1. Delivery device (100, 200) for delivering a game object, comprising:
a drive (101, 201) having a drive body (103, 111, 203, 205) for driving the game object according to a controllable drive characteristic; and
a control device (107) for controlling the controllable drive characteristic as a function of at least one performance-physiological parameter,
wherein the performance physiological parameter is an actual parameter of a player, wherein the control device is configured to control the controllable drive characteristic of the drive as a function of the performance physiological actual parameter and a performance-physiological target parameter, wherein the control of the drive characteristic of the drive body is only performed when the target-to-actual value difference reaches or exceeds a predetermined or adjustable threshold value.

2. Delivery device according to claim 1, wherein the control device (107) is configured to control the controllable drive characteristic as a function of at least one of the following performance physiological parameters: vital parameters, in particular heart rate, respiratory rate, oxygen concentration, blood sugar value, blood pressure, skin conductance, myoelectric activity, brain electrical activity, or biomechanical parameters, in particular a time parameter, a biokinematic parameter or a biodynamic parameter.

3. Delivery device according to one of the preceding claims, wherein the performance physiological parameter comprises a performance-physiological target parameter or a physiological-physiological actual parameter, in particular a performance-physiological target parameter or a performance-physiological actual parameter of a player.

4. Delivery device according to one of the preceding claims, comprising a communication interface (113) which is configured to receive the performance physiological parameter or information about the performance physiological parameter, in particular at least one measured value of the performance physiological parameter, via a communication network.

5. Delivery device according to one of the preceding claims, comprising a memory for storing a plurality of selectable performance physiological parameters, in particular a plurality of target parameters, wherein the performance physiological parameter, in particular as a performance-physiological target parameter, is selectable by means of a graphical user interface.

6. Delivery device according to one of the preceding claims, wherein the drive body is a transversely displaceable drive body and wherein the drive characteristic is a linear drive characteristic, or wherein the drive body (103, 111, 203, 205) is a rotatable drive body and wherein the drive characteristic is a rotation characteristic, or wherein the drive body (103, 111 , 203, 205) is a rotatable drive body and wherein the drive further comprises a transversely displaceable drive body, and wherein the drive characteristic comprises a linear drive characteristic of the transversely displaceable drive body and a rotation characteristic of the rotatable drive body (103, 111, 203, 205).

7. Delivery device according to one of the preceding claims, wherein the drive body (103, 111, 203, 205) is a rotatable drive body (103, 111, 203, 205), and wherein the control device (107) is configured to control at least one of the following rotation parameters of the drive body (103 , 111, 203, 205) for controlling the controllable drive characteristic: rotational speed, direction of rotation, rotational acceleration, duration of rotation, tilt of a rotation axis, tilt of a rotation axis with respect to a perpendicular axis, tilt of the drive body.

8. Delivery device according to one of the preceding claims, wherein the drive body (103, 111, 203, 205) is a rotatable (103, 111, 203, 205) drive body, wherein the drive (101, 201) comprises a second rotatable drive body (103, 111, 203, 205), and wherein the game object is drivable by means of the rotatable drive body and the second rotatable drive body (103, 111, 203, 205).

9. Delivery device according to claim 8, wherein the second rotatable drive body (103, 111, 203, 205) comprises a second controllable drive characteristic, in particular a rotation characteristic, and wherein the control device (107) is configured to control the second drive characteristic as a function of the performance physiological parameter.

10. Delivery device according to one of the preceding claims, wherein the drive (101, 201) comprises a wall, on which the game object can be guided or unrolled, and wherein the game object can be introduced between the drive body (103, 111, 203, 205) and the wall.

11. Delivery device according to one of the preceding claims, wherein the respective drive body (103, 111, 203, 205) comprises a drive pulley or a drive ball or a drive cone.

12. Delivery device according to one of the preceding claims, wherein the respective drive body (103, 111, 203, 205) is rotatable, in particular rotatably mounted around an axis of rotation lying outside the respective drive body (103, 111, 203, 205), and wherein the control device (107) is configured to rotate the drive body (103, 111, 203, 205) as a function of the performance physiological parameter or as a function of a delivery direction of the game object, in particular of a randomly selectable or an adjustable delivery direction.

13. Delivery device according to one of the preceding claims 1 to 6, wherein the drive body is a transversely displaceable drive body, which is configured to exert a shock pulse on the game object.

14. Delivery device according to claim 13, wherein the drive body is a transversely displaceable drive body, wherein the drive characteristic is a linear drive characteristic, and wherein the control device (107) is configured to control at least one of the following drive parameters of the drive body for controlling the controllable drive characteristic: pulse, acceleration, lateral offset with respect to the game object, angular offset with respect to the game object, or tilt of the drive body with respect to a perpendicular axis.

15. Delivery device according to one of the preceding claims, which is configured to deliver a plurality of game objects in a predetermined, in particular adjustable, time interval.

16. Delivery device according to one of the preceding claims, which is configured to generate a delivery indication signal, in particular an acoustic or optical delivery indication signal, before or with delivery of the game object.

17. Delivery device according to one of the preceding claims, wherein the game object is a ball, in particular a football, an American football, a rugby ball, a basketball, a baseball, a volleyball, a handball, a golf ball, a cricket ball, a polo ball, a ping-pong ball, or where the game object is a hockey puck.

18. Method for delivering a game object by means of a drive body which comprises a rotatable drive body for driving the game object according to a controllable drive characteristic, comprising:
controlling the controllable drive characteristic as a function of at least one performance-physiological parameter,
wherein the performance physiological parameter is an actual parameter of a player, wherein the controllable drive characteristic of the drive is controlled as a function of the performance physiological actual parameter and a performance-physiological target parameter, wherein the control of the drive characteristic of the drive body is only performed when the target-to-actual value difference reaches or exceeds a predetermined or adjustable threshold value.

19. Training system, comprising:
a measuring device (301) for detecting at least one performance-physiological parameter of a player; and
the delivery device (100, 200) for delivering a game object according to one of claims 1 to 17 as a function of the performance physiological parameter of the player.

## Revendications

1. Dispositif lanceur (100, 200) destiné à lancer un objet de jeu, comprenant :
un système de propulsion (101, 201) comportant un corps de propulsion (103, 111, 203, 205) destiné à propulser l'objet de jeu conformément à une caractéristique de propulsion réglable ; et
un dispositif de réglage (107) destiné à régler la caractéristique de propulsion réglable en fonction d'au moins un paramètre physiologique de performance,
dans lequel le paramètre physiologique de performance est un paramètre réel d'un joueur, dans lequel le dispositif de réglage est conçu pour régler la caractéristique de propulsion réglable du système de propulsion en fonction du paramètre physiologique de performance réel et d'un paramètre physiologique de performance nominal, dans lequel le réglage de la caractéristique de propulsion du corps de propulsion n'est effectué que lorsque la différence entre les valeurs nominale et réelle atteint ou dépasse une valeur de seuil prédéterminée ou réglable.

2. Dispositif lanceur selon la revendication 1, dans lequel le dispositif de réglage (107) est conçu pour régler la caractéristique de propulsion réglable en fonction d'au moins l'un des paramètres physiologiques de performance suivants : des paramètres vitaux, notamment la fréquence cardiaque, la fréquence respiratoire, la concentration d'oxygène, la glycémie, la tension artérielle, la résistance à la conductance de la peau, l'activité myoélectrique, l'activité électrique cérébrale, ou des paramètres biomécaniques, notamment un paramètre temporel, un paramètre biocinématique ou un paramètre biodynamique.

3. Dispositif lanceur selon l'une des revendications précédentes, dans lequel le paramètre physiologique de performance comprend un paramètre physiologique de performance nominal ou un paramètre physiologique de performance réel, notamment un paramètre physiologique de performance nominal ou un paramètre physiologique de performance réel d'un joueur.

4. Dispositif lanceur selon l'une des revendications précédentes, comportant une interface de communication (113) destinée à recevoir le paramètre physiologique de performance ou des informations concernant le paramètre physiologique de performance, notamment au moins une valeur de mesure du paramètre physiologique de performance, par l'intermédiaire d'un réseau de communication.

5. Dispositif lanceur selon l'une des revendications précédentes, comportant une mémoire destinée à stocker une pluralité de paramètres physiologiques de performance sélectionnables, notamment une pluralité de paramètres nominaux,
dans lequel le paramètre physiologique de performance, notamment en tant que paramètre physiologique de performance nominal, peut être sélectionné au moyen d'une interface utilisateur graphique.

6. Dispositif lanceur selon l'une des revendications précédentes, dans lequel le corps de propulsion est un corps de propulsion pouvant être déplacé transversalement et dans lequel la caractéristique de propulsion est une caractéristique de propulsion linéaire, ou dans lequel le corps de propulsion (103, 111, 203, 205) est un corps de propulsion rotatif et dans lequel la caractéristique de propulsion est une caractéristique de rotation, ou dans lequel le corps de propulsion (103, 111, 203, 205) est un corps de propulsion rotatif, et dans lequel le système de propulsion comprend en outre un corps de propulsion pouvant être déplacé transversalement, et dans lequel la caractéristique de propulsion comprend une caractéristique de propulsion linéaire du corps de propulsion pouvant être déplacé transversalement et une caractéristique de rotation du corps de propulsion rotatif (103, 111, 203, 205).

7. Dispositif lanceur selon l'une des revendications précédentes, dans lequel le corps de propulsion (103, 111, 203, 205) est un corps de propulsion rotatif (103, 111, 203, 205), et
dans lequel le dispositif de réglage (107) est conçu pour régler au moins l'un des paramètres de rotation suivants du corps de propulsion (103, 111, 203, 205) afin de régler la caractéristique de propulsion réglable : la vitesse de rotation, le sens de rotation, l'accélération de la rotation, la durée de rotation, l'inclinaison d'un axe de rotation, l'inclinaison d'un axe de rotation par rapport à un fil à plomb vertical, l'inclinaison du corps de propulsion.

8. Dispositif lanceur selon l'une des revendications précédentes, dans lequel le corps de propulsion (103, 111, 203, 205) est un corps de propulsion rotatif (103, 111, 203, 205), dans lequel le système de propulsion (101, 201) comprend un second corps de propulsion rotatif (103, 111, 203, 205), et dans lequel l'objet de jeu peut être propulsé au moyen du corps de propulsion rotatif et du second corps de propulsion rotatif (103, 111, 203, 205).

9. Dispositif lanceur selon la revendication 8, dans lequel le second corps de propulsion rotatif (103, 40, 111, 203, 205) présente une seconde caractéristique de propulsion réglable, notamment une caractéristique de rotation, et dans lequel le dispositif de réglage (107) est conçu pour régler la seconde caractéristique de propulsion en fonction du paramètre physiologique de performance.

10. Dispositif lanceur selon l'une des revendications précédentes, dans lequel le système de propulsion (101, 201) comprend une paroi sur laquelle l'objet de jeu peut être guidé ou enroulé, et dans lequel l'objet de jeu peut être inséré entre le corps de propulsion (103, 111, 203, 205) et la paroi.

11. Dispositif lanceur selon l'une des revendications précédentes, dans lequel le corps de propulsion respectif (103, 111, 203, 205) comprend un disque de propulsion ou une bille de propulsion ou un cône de propulsion.

12. Dispositif lanceur selon l'une des revendications précédentes, dans lequel le corps de propulsion respectif (103, 111, 203, 205) est monté de manière rotative, notamment autour d'un axe de rotation situé en dehors du corps de propulsion respectif (103, 111, 203, 205), et dans lequel le dispositif de réglage (107) est conçu pour mettre en rotation le corps de propulsion (103, 111, 203, 205) en fonction du paramètre physiologique de performance ou en fonction d'une direction de lancement de l'objet de jeu, notamment d'une direction de lancement sélectionnable aléatoirement ou réglable.

13. Dispositif lanceur selon l'une des revendications 1 à 6 précédentes, dans lequel le corps de propulsion est un corps de propulsion pouvant être déplacé transversalement, qui est conçu pour appliquer une impulsion de choc à l'objet de jeu.

14. Dispositif lanceur selon la revendication 13, dans lequel le corps de propulsion est un corps de propulsion pouvant être déplacé transversalement, dans lequel la caractéristique de propulsion est une caractéristique de propulsion linéaire, et dans lequel le dispositif de réglage (107) est conçu pour régler au moins l'un des paramètres de propulsion suivants du corps de propulsion afin de régler la caractéristique de propulsion réglable : une impulsion, une accélération, un décalage latéral par rapport à l'objet de jeu, un décalage angulaire par rapport à l'objet de jeu, ou une inclinaison du corps de propulsion par rapport à un fil à plomb vertical.

15. Dispositif lanceur selon l'une des revendications précédentes, qui est conçu pour lancer une pluralité d'objets de jeu à un intervalle de temps prédéterminé, notamment réglable.

16. Dispositif lanceur selon l'une des revendications précédentes, qui est conçu pour générer un signal d'indication de lancement, notamment un signal acoustique ou optique, avant ou au moment du lancement de l'objet de jeu.

17. Dispositif lanceur selon l'une des revendications précédentes, dans lequel l'objet de jeu est un ballon/une balle, notamment un ballon de football, un ballon de football américain, un ballon de rugby, un ballon de basket-ball, une balle de baseball, un ballon de volley-ball, un ballon de handball, une balle de golf, une balle de cricket, une balle de polo, une balle de tennis de table ou dans lequel l'objet de jeu est un palet de hockey.

18. Procédé de lancement d'un objet de jeu au moyen d'un corps de propulsion comprenant un corps de propulsion rotatif destiné à propulser l'objet de jeu en fonction d'une caractéristique de propulsion réglable, comprenant :
le réglage de la caractéristique de propulsion réglable en fonction d'au moins un paramètre physiologique de performance,
dans lequel le paramètre physiologique de performance est un paramètre réel d'un joueur, dans lequel la caractéristique de propulsion réglable du système de propulsion est réglée en fonction du paramètre physiologique de performance réel et d'un paramètre physiologique de performance nominal, dans lequel le réglage de la caractéristique de propulsion du corps de propulsion n'est effectué que lorsque la différence entre les valeurs nominale et réelle atteint ou dépasse une valeur de seuil prédéterminée ou réglable.

19. Système de propulsion, comprenant :
un dispositif de mesure (301) destiné à détecter au moins un paramètre physiologique de performance d'un joueur ; et
le dispositif lanceur (100, 200) selon l'une des revendications 1 à 17, destiné à lancer un objet de jeu en fonction du paramètre physiologique de performance du joueur.
